# EUROPEAN PATENT APPLICATION

(11) **EP 2 233 079 A2**
(43) Date of publication of application: **29.09.2010**
(21) Application number: 10157122.2
(22) Date of filing: 19.03.2010
(51) Int. Cl.: A61B 8/12, H05K 9/00, A61L 29/10, C23C 14/20, A61M 25/00

(54) **Ultrasound catheter housing with electromagnetic shielding properties and methods of manufacture**

(30) Priority: 25.03.2009 US 410916
(71) Applicant: GENERAL ELECTRIC COMPANY, Schenectady, NY 12345 (US)
(72) Inventor: Buckley, Donald Joseph, Schenectady, NY 12304 (US); Wildes, Douglas Glenn, Ballston Lake, NY 12027 (US); Lee, Warren, Niskayuna, NY 12309 (US); Griffin, Weston Blaine, Niskayuna, NY 12309 (US)
(74) Representative: Illingworth-Law, William Illingworth

(57) **Abstract**

An ultrasound catheter housing (10) with electromagnetic shielding properties and methods of manufacturing is provided. The ultrasound catheter housing (10) comprises a an inner thin wall polymer tube (12) extruded using an ultrasonically transparent polymer, a thin metalized layer (14) deposited on the outer surface of the inner tube, and an outer thin wall polymer tube (16), which may be the same or a different ultrasonically transparent material. In another embodiment an ultrasound catheter (18) comprising the ultrasound catheter housing (10) is provided.

## Description

### BACKGROUND OF THE INVENTION

Background noise is a consideration in all imaging modalities. Excessive noise may limit sensitivity and resolution of the image. This is the case in intracardiac echo (ICE) ultrasonic imaging, where the proximity of a high frequency noise source, such as an RF ablation catheter or other electrosurgical device, will degrade the images obtained with the ICE catheter. Other sources of electromagnetic interference are the magnetic field of the motor driving transducer motion in the 4D ICE configuration, and EM fields associated with positioning systems. To limit the noise signal entering the transducer and its associated cabling, an electromagnetic shield is required. Such a shield must be ultrasonically transparent, but opaque (or nearly so) to electromagnetic radiation in the expected frequency regimes.

### BRIEF DESCRIPTION OF THE INVENTION

This invention describes a multilayer ultrasound catheter housing comprising metal layers and polymer layers in order to provide electromagnetic or low frequency magnetic shielding with minimum impact on the acoustic performance and tracking of the ultrasound catheter.

In an embodiment, an ultrasound catheter housing with electromagnetic shielding properties includes an ultrasonically transparent inner polymer tube; a metal layer deposited on the outer surface of the inner polymer tube; an ultrasonically transparent outer tube deposited on the outer surface of the metal layer; and wherein the metal layer is embedded between the inner polymer tube and the outer polymer tube.

In another embodiment an ultrasound catheter comprises a flexible catheter housing defining a distal end; a transducer array disposed within the catheter housing; and a motor coupled with the transducer array. The catheter housing comprises an ultrasonically transparent inner polymer tube; a metal layer deposited on the outer surface of the inner polymer tube; an ultrasonically transparent outer tube deposited on the outer surface of the metal layer. The motor is configured to rotate the transducer array in order to image a three-dimensional volume.

In another embodiment, a method of manufacturing a catheter housing with electromagnetic shielding is provided and includes the steps of creating a polymer inner layer supported on an inner form or mandrel; coating the polymer layer with metal; adding a polymer outer layer over the metal layer; and removing the inner form or mandrel.

Various other features, objects, and advantages of the invention will be made apparent to those skilled in the art from the accompanying drawings and detailed description thereof.

### BRIEF DESCRIPTION OF THE DRAWINGS

FIG. 1 is a schematic representation of an ultrasound catheter housing having a three layer sandwich structure.

FIG. 2 is a partially cutaway schematic illustration of an ICE catheter for use with an embodiment of the invention.

FIG. 3 is a flow diagram illustrating steps in manufacturing an ultrasound catheter housing

### DETAILED DESCRIPTION OF THE INVENTION

This invention describes materials and processes for manufacture of an ultrasound catheter housing having electromagnetic (EM) shielding properties.

Operation of a surgical navigational device comprising an ultrasound catheter in the vicinity of sources of electrical noise, and particularly radio-frequency (RF) noise sources, can result in excessive noise background. This may result in obscuring detail and limiting contrast and resolution during ultrasound imaging. Electromagnetic (EM) noise may be generated by operation of the ultrasound catheter's motor. It may also be generated by operating the ultrasound catheter in the vicinity of, or in collaboration with other electronic medical devices such as RF ablation devices, Bovie knives, electro surgery devices, or sensitive electrical circuits such as intracardiac electrocardiogram (ECG) leads. EM interference is a concern in ultrasound catheters containing transducer arrays that move within an outer housing, for example multiplane transesophageal echo (TEE) probes, mechanical 4D (three dimensional volume imaging with time as a 4^{th} parameter) probes, and 4D intracardiac echocardiography (ICE) catheters.

Reduction of EM noise pickup by the ultrasound catheter is essential to optimize the ultrasound image produced by the transducer array and permit full use of its resolution and contrast, and to reduce artifacts in the ultrasound images. For instance, it is important for an ICE catheter to be able to generate low-noise images simultaneously when used with operation of an ablation catheter; the ablation catheter tip is a potent source of RF noise. Reduction of EM noise, including RF noise, is essential to preserve signal integrity and diagnostic information in other devices operating near the ultrasound probe. In addition to shielding the ultrasound catheter from external noise, it may also be desirable to keep ultrasound signals (electrical) and motor signals (magnetic) from interfering with other sensitive devices and signals, for example ECG .

To reduce or eliminate EM noise detected by an ultrasound catheter, the ultrasound tip housing can be a sandwich structure consisting of three components; an inner thin wall polymer tube extruded using an ultrasonically transparent polymer, a thin metalized layer deposited on the outer surface of the inner tube, and an outer thin wall polymer tube, which may be the same or a different ultrasonically transparent material employed as the inner layer, which is extruded over the metalized layer. This results in a sandwich structure with an embedded metal layer between two layers of polymer.

The embedded metalized layer, when suitably grounded, may act as a shield against electrical noise and especially RF radiation. The polymer layers provide protection to the metalized layer against abrasion and leaching of metal into the blood stream during use. The metal layer must be of sufficient thickness to provide an effective Faraday cage around the ultrasound catheter and its associated cabling without reducing ultrasound transmission significantly, or introducing reflections into the signal. The metal layer must also adhere to both the inner and outer polymer tubing without delaminating from either, which would result in an air gap and near complete reflection of ultrasound energy emitted by the ultrasound catheter.

In one embodiment, the metal layer may be deposited by electroless plating. In another embodiment the metal layer may be deposited by sputter coating or by ion beam assisted deposition or by some other appropriate process. The metal layer may consist of copper, aluminum, gold, silver, combinations thereof, or other metals having high electrical conductivity at RF frequencies. A metal having high electrical conductivity at RF frequencies may be defined as a metal having electrical conductivity, as measured in units of siemens per meter (S·m⁻¹), greater than 20 x10⁶.

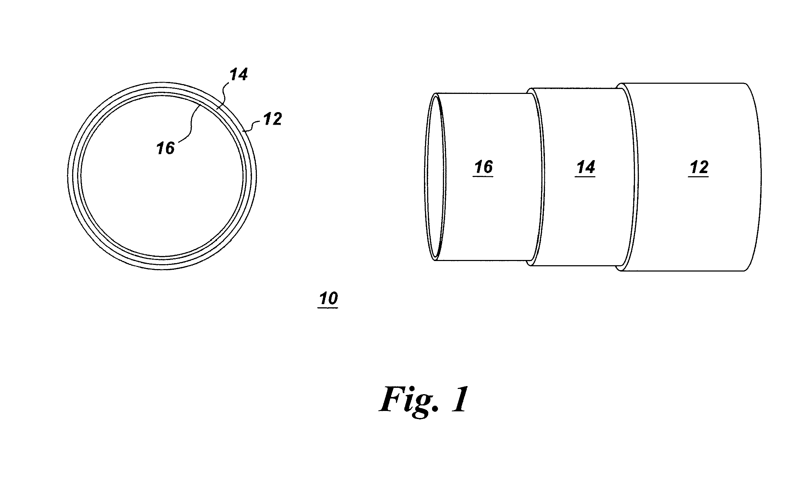

The polymer layers may be of sufficient thickness to resist abrasion and normal wear without exposing the metal layer. The polymer layers also must sustain the mechanical loads imposed on the catheter tip during to prevent bending, buckling or kinking in a manner, which may interfere with rotation of the transducer array. In certain embodiments, the metal layer may be between 0.1-10 microns in thickness, however the thickness may be smaller or larger depending on the catheter device. For example an intravascular ultrasound (IVUS) catheter may have an overall diameter of less than 3 Fr (1mm) while an abdominal aortic aneurysm (AAA) repair device may have an overall diameter of greater than 22 Fr (7mm). In one embodiment the metal layer is between 0.8 and 1.5 microns in thickness. Referring further to FIG. 1, the three-layer sandwich structure may have an inner diameter of between 600 microns to 7.6 millimeters and an outer diameter between 1000 microns to 8 millimeters.

As shown in FIG. 1 an ultrasound catheter housing 10 may include an inner polymer layer 12, a metal layer 14, and an outer polymer layer 16. In regions where the ultrasound catheter housing is in the acoustic path, the metal layer 14 may be from 0.1 to 10 microns in thickness, preferably from 0.8 to1.5 microns in thickness, and have high conductivity. Suitable metals include, but are not limited to, copper, aluminum, gold, and silver. The metal layer provides EM noise reduction, including RF noise reduction, without significantly impairing ultrasound performance. The metal layer 14 is sufficiently thin to permit passage of most ultrasound energy from and to the ultrasound catheter. The metal layer may also be connected via a low-impedance conductor to a suitable ground.

In regions where low frequency magnetic shielding is needed, a thicker metal layer of a high-permeability metal may be required. For example, a particular small brushless DC micro-motor, which may be used in the ultrasound catheter, produces an external field of approximately 100 Gauss. In one embodiment to achieve significant shielding close to the motor (0.3 mm gap) without saturation, a 0.1 mm thickness of a high-permeability magnetic shielding alloy may be used in addition to the metal layer 14. High-permeability magnetic shielding alloy includes alloys composed primarily of nickel. The remainder of the material includes iron, molybdenum, chromium, copper, and combinations thereof. The high permeability materials may act to absorb and redirect magnetic flux. In certain embodiments high permeability alloys such as CO-NETIC® from Magnetic Shield Corp may be used, or high permittivity alloys known generically as "mu metal"

In certain embodiments, various organic or oxide or metal (Ni, Cr, Ti) layers may be applied between the metal layer and the polymer layers in order to improve adhesion. The metal layers may be a continuous layer. In other embodiments, the outer surface of the inner polymer layer may be activated by chemical, plasma or corona treatment, and the outer metal layer surface may be treated with organometallic compounds, or other chemical treatment, in both instances with the aim to improve adhesion.

In other embodiments, the metal layer may have one or more small openings, either to enhance adhesion between the inner and outer polymer layers or to allow one to see through the shield to inspect the contents of the housing. In certain embodiments, the metal layer may be a metal mesh having small and separate openings; in other embodiments the opening may comprise a narrow non-metalized strip, positioned in a straight line or spiraling around the inner polymer layer. The intervening layer between the polymer layers and the metal layer may also be a "tie" layer; a bifunctional material with functional groups which bond well to the polymer and metal, respectively.

The polymer used in layers 12 and 16 may have acoustic properties near those of the acoustic coupling fluid, which may be water, saline solution or propylene glycol. In one embodiment the polymers have sound velocities in the range 1.0 to 3.0 millimeters per microsecond, and acoustic impedances in the range of 1.0 to3.0 MegaRayls

In certain embodiments the inner and outer polymer layer are comprised of the same material. In other embodiments the inner layer may be of a material that can be made very thin without pinholes (e.g. polyester and polyester film such as Mylar^{®} available from DuPont) and the outer layer may be a biocompatible material with good acoustic and structural properties such as polymethylpentene, which is available as Mitsui TPX^{™}.

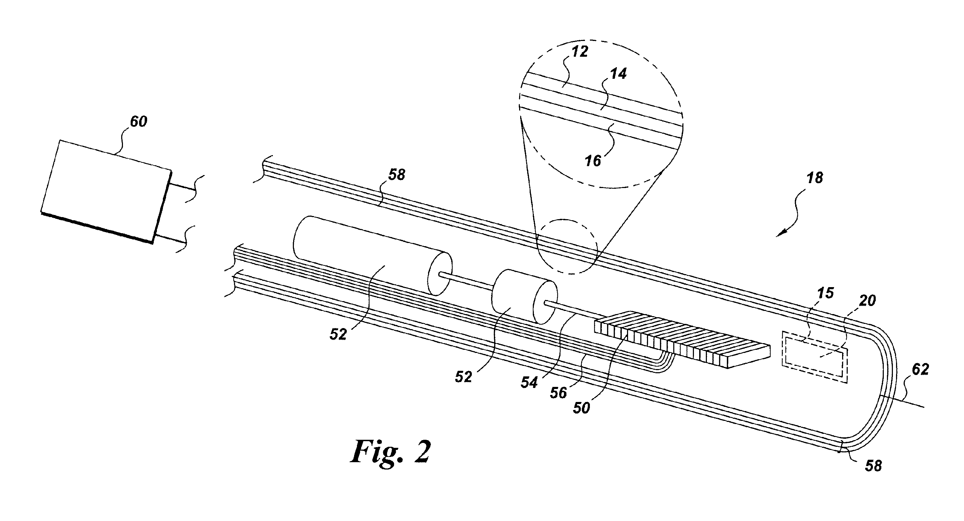

Referring to Figure 2, an illustration of an ICE catheter 18 is shown which may incorporate the catheter housing described above. It should be appreciated that the ICE catheter 18 is described for illustrative purposes, and that any catheter system adapted to retain an ultrasound imaging device may alternatively be implemented in place of the ICE catheter 18.

The ICE catheter 18 comprises a transducer array 50, a motor and gearbox 52, which may be internal or external to the space-critical environment, a drive shaft 54, and an interconnect 56. The ICE catheter 18 further includes a catheter housing 58 enclosing the transducer array 50, motor and gear box 52, interconnect 56 and drive shaft 54. In the depicted embodiment, the transducer array 50 is mounted on drive shaft 54 and the transducer array 50 is rotatable with the drive shaft 54. Motor controller 60 and motor 52 control the rotational motion of the transducer array 50. Interconnect 56 refers to, for example, cables and other connections coupling the transducer array 50 with the ICE imaging device 32 (not shown) for use in receiving and transmitting signals. In an embodiment, interconnect 56 is configured to reduce its respective torque load on the transducer array 50 and motor 52.

The catheter housing 58 is of a material, size and shape adaptable for internal imaging applications and insertion into regions of interest. According to the embodiment depicted in Figure 2, the catheter housing 58 is generally cylindrical defining a longitudinal axis 62.The catheter housing 58, or at least the portion that intersects the ultrasound imaging volume, is acoustically transparent, e.g. low attenuation and scattering, acoustic impedance near that of blood and tissue (Z between 1.0 to3.0 MegaRayls). In the embodiment shown, the entire catheter housing is composed of a three-layer sandwich structure wherein the metal layer 14 is encased by an outer polymer layer 12 and an inner polymer layer 16. In other embodiments, the catheter housing may have an area comprising the three-layer sandwich structure and an area comprising a single polymer layer. The space between the transducer and the housing can be filled with an acoustic coupling fluid (not shown), e.g., water or propylene glycol.

According to one embodiment, the transducer array 50 is a 64-element one-dimensional array having 0.110 mm azimuth pitch, 2.5 mm elevation and 6.5 MHz center frequency. The elements of the transducer array 50 are electronically phased in order to acquire a sector image parallel to the longitudinal axis 62 of the catheter housing 58. The transducer array 50 is mechanically rotated about the longitudinal axis 62 to image a three-dimensional volume. The transducer array 50 captures a plurality of two-dimensional images as it is being rotated. The plurality of two-dimensional images are transmitted to the ICE imaging device 32 (not shown) which is configured to sequentially assemble the two-dimensional images in order to produce a three-dimensional image.

The motor controller 60 can regulate the rate at which the transducer array 50 is rotated about the longitudinal axis 62. The transducer array 50 can be rotated relatively slowly to produce a 3D image, or relatively quickly to produce a generally real time 3D image (i.e., a 4D image). The motor controller 60 is also operable to vary the direction of rotation to produce an oscillatory transducer array motion. In this manner, the range of motion and imaged volume are restricted such that the transducer array 50 can focus on imaging a specific region and can update the 3D image of that region more frequently, thereby providing a real-time 3D, or 4D, image.

Referring further to FIG. 2, the ICE catheter 18 includes an integrally attached tracking element 20 disposed within the catheter housing 58. The integrally attached tracking element 20 is adapted to estimate the position and orientation of the ICE catheter 18. While the tracking element 20 is depicted as comprising a field sensor 15 in accordance with one embodiment, it should be appreciated that the tracking element 20 may alternatively comprise a field generator 21 (not shown) similar to the field sensor 15.

The field sensor 15 may comprise two or more coils adapted to track the ICE catheter 18 with six degrees of freedom. For purposes of this disclosure, the six degrees of freedom refer to the position along each of the three primary X, Y and Z axes as well as orientation or degree of rotation about each of the three primary axes (i.e., yaw, pitch and roll). The field sensor 15 may define a variety of different coil configurations. According to one embodiment, the field sensor 15 comprises two generally co-located micro-coils. According to another embodiment, the field sensor 15 comprises three generally orthogonal coils defining an industry-standard-coil-architecture (ISCA) type configuration.

The tracking element 20 may be positioned immediately adjacent to the distal end of the catheter housing 58, away from the motor. For purposes of this disclosure, the term "immediately adjacent" refers to the depicted arrangement wherein there are no other components disposed between the tracking element 20 and the distal end.. In other embodiments, the tracking element 20 may be positioned in other locations within the catheter housing due to other constraints, for example insufficient space for the tracking element cables to pass by the transducer array.

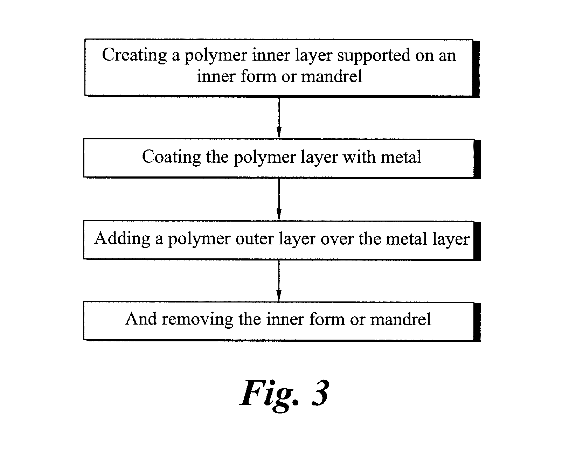

FIG. 3 illustrates one embodiment for manufacturing a shielded catheter housing. The process comprises creating a polymer inner layer supported on an inner form or mandrel, coating the polymer layer with metal, adding a polymer outer layer over the metal layer, and removing the inner form or mandrel.

In an embodiment, a high-permeability magnetic shield material may be added in one or more selected regions of the catheter housing. The magnetic shield material may be added by forming or wrapping the shield material around the metal layer. In another embodiment, the shield material may be added by forming or wrapping the shield material directly around a section of the polymer inner layer still exposed and not coated with metal.

The polymer inner layer may have a thickness of 10 to 200 microns and be shaped to facilitate usage of the ultrasound catheter; for example a tubular construction. The metal layer may have a thickness of 0.1 to 10 microns and be comprised of a highly conductive material such as, but not limited to copper, aluminum, gold, or silver. The metal layer may be applied by various techniques including sputtering, evaporating, ion beam assisted deposition, electroplating, or electroless plating. The polymer outer layer may be applied using extrusion molding or dip coating wherein the thickness of the polymer outer layer is from 100 to 260 microns.

In certain embodiments, the inner form or mandrel may be removed by sliding the shielded housing off the mandrel. In other embodiments, the mandrel may be removed by chemically etching the mandrel away from the inner surface of the shielded housing.

The outer polymer layer over the metal layer improves biocompatibility and provides an insulating dielectric layer for electrical safety (dielectric breakdown; leakage current). An inner polymer layer protects the metal layer from corrosion and wear and provides electrical isolation between the metal layer and the internal components of the ultrasound catheter. Alternatively, a metal housing with an acoustic window could be constructed, and subsequently coated with a polymer layer to improve biocompatibility. A mandrel could be used to support the polymer layer over the acoustic window as it is extruded over the metal housing. Such a structure could provide EM shielding as well as structural integrity. In another embodiment the metal layer may be formed from a polymer composite containing metal filler.

The ultrasound catheter housing described above may allow for ultrasound imaging simultaneous with other clinical procedures requiring EM shielding such as radio frequency (RF) ablation, electrosurgery, ECG monitoring, or tracking the position of devices. Without adequate shielding, external noise sources would severely disrupt the ultrasound image (typically a bright "searchlight" in the center of the image, or bright noise throughout the image), preventing the visualization of all but the most high-contrast anatomy. Without adequate shielding, the motor driving the oscillating motion of the transducer would interfere with position tracking, ECG monitoring, and other sensors. It would therefore be necessary to turn off RF ablation and other RF noise sources during ultrasound imaging, and turn off 4D motion when monitoring ECG signals or device positions. Proper shielding in the catheter housing allows the ultrasound probe to provide necessary 2D and 4D image quality as measured by contrast, resolution and penetration during simultaneous operation with other devices. The shielding allows the 4D ultrasound probe to become a useful, unrestricted tool for visualization of anatomy and clinical devices and procedures.

The invention may be embodied in other specific forms without departing from the spirit or essential characteristics thereof. The foregoing embodiments are therefore to be considered in all respects as illustrative rather than limiting on the invention described herein. The scope of the invention is thus indicated by the appended claims rather than by the foregoing description, and all changes that come within the meaning and range of equivalency of the claims are therefore intended to be embraced therein.

Aspects of the present invention are defined in the following numbered clauses:
1. An ultrasound catheter housing with electromagnetic shielding properties comprising:
   an ultrasonically transparent inner polymer tube;
   a metal layer deposited on the outer surface of the inner polymer tube;
   an ultrasonically transparent outer tube deposited on the outer surface of the metal layer; and wherein the metal layer is embedded between the inner polymer tube and the outer polymer tube.
2. The ultrasound catheter housing of clause 1 wherein the metal layer comprises copper, aluminum, gold, silver, or combinations thereof.
3. The ultrasound catheter housing of clause 1 or clause 2 wherein the metal layer has a thickness between 0.1 and 10 microns.
4. The ultrasound catheter housing of any one of the preceding clauses wherein the metal layer has one or more openings.
5. The ultrasound catheter housing of any one of the preceding clauses wherein the inner polymer tube and the outer polymer tube have sound velocities in the range 1.0 to 3.0 millimeters per microsecond, and acoustic impedances in the range of 1.0 to3.0 MegaRayls.
6. The ultrasound catheter housing of any one of the preceding clauses wherein the inner polymer tube and the outer polymer tube independently are comprised of polyester or polymethylpentene.
7. The ultrasound catheter housing of any one of the preceding clauses wherein an adhesion layer is situated between the metal layer and at least one of the inner polymer tube and the outer polymer tube.
8. The ultrasound catheter housing of clause 7 wherein the adhesion layer is comprised of an organic oxide or a metal oxide.
9. The ultrasound catheter housing of any one of the preceding clauses wherein the metal layer comprises a high permeability magnetic shielding alloy.
10. An ultrasound catheter comprising:
   a catheter housing said catheter housing comprising an ultrasonically transparent inner polymer tube, a metal layer deposited on the outer surface of the inner polymer tube, an ultrasonically transparent outer tube deposited on the outer surface of the metal layer, and whereby the metal layer is embedded between the inner polymer tube and the outer polymer tube;
   a transducer array disposed at least partially within the catheter housing;
   a motor coupled with the transducer array, said motor being configured to rotate the transducer array in order to image a three-dimensional volume; and
   a tracking element adapted to provide an estimate of a position and/or orientation of the distal end of the catheter housing, said tracking element disposed within the catheter housing.
11. The ultrasound catheter of clause 10, wherein the tracking element comprises at least one of a magnetic field sensor or a magnetic field generator.
12. The ultrasound catheter of clause 10 or clause 11 wherein the metal layer comprises copper, aluminum, gold, silver, or combinations thereof
13. The ultrasound catheter of any one of clauses 10 to 12 wherein the metal layer has a thickness between 0.1 and 10 microns.
14. The ultrasound catheter of any one of clauses 10 to 13 wherein the metal layer has one or more openings.
15. The ultrasound catheter of any one of clauses 10 to 14 wherein the inner polymer tube and the outer polymer tube have sound velocities in the range 1.0 to 3.0 millimeters per microsecond, and acoustic impedances in the range of 1.0 to3.0 MegaRayls.
16. The ultrasound catheter of any one of clauses 10 to 15 wherein the inner polymer tube and the outer polymer tube independently are comprised of polyester or polymethylpentene.
17. The ultrasound catheter of any one of clauses 10 to 16 wherein an adhesion layer is situated between the metal layer and at least one of the inner polymer tube and the outer polymer tube.
18. The ultrasound catheter of clause 17 wherein the adhesion layer is comprised of an organic oxide or a metal oxide.
19. The ultrasound catheter housing of any one of clauses 10 to 16 wherein the metal layer comprises a high permeability magnetic shielding alloy.
20. The ultrasound catheter of any one of clauses 10 to 19 wherein the catheter is an intracardiac echocardiography (ICE) catheter.
21. A method of manufacturing an ultrasound catheter housing comprising:
   creating a polymer inner layer supported on an inner form or mandrel;
   coating the polymer layer with metal;
   adding a polymer outer layer over the metal layer; and
   removing the inner form or mandrel.

## Claims

1. An ultrasound catheter housing with electromagnetic shielding properties comprising:
an ultrasonically transparent inner polymer tube;
a metal layer deposited on the outer surface of the inner polymer tube;
an ultrasonically transparent outer tube deposited on the outer surface of the metal layer; and wherein the metal layer is embedded between the inner polymer tube and the outer polymer tube.

2. The ultrasound catheter housing of claim 1, wherein the metal layer comprises copper, aluminum, gold, silver, or combinations thereof.

3. The ultrasound catheter housing of claim 1 or claim 2, wherein the metal layer has a thickness between 0.5 and 5 microns.

4. The ultrasound catheter housing of any one of the preceding claims, wherein the inner polymer tube and the outer polymer tube have sound velocities in the range 1.0 to 3.0 millimeters per microsecond, and acoustic impedances in the range of 1.0 to3.0 MegaRayls.

5. The ultrasound catheter housing of any one of the preceding claims, wherein an adhesion layer is situated between the metal layer and at least one of the inner polymer tube and the outer polymer tube.

6. The ultrasound catheter housing of any one of the preceding claims, further comprising a high permeability magnetic shielding alloy adhered to the metal layer.

7. An ultrasound catheter comprising:
a flexible catheter housing said catheter housing comprising an ultrasonically transparent inner polymer tube, a metal layer deposited on the outer surface of the inner polymer tube, an ultrasonically transparent outer tube deposited on the outer surface of the metal layer, and whereby the metal layer is embedded between the inner polymer tube and the outer polymer tube;
a transducer array disposed at least partially within the catheter housing;
a motor coupled with the transducer array, said motor being configured to rotate the transducer array in order to image a three-dimensional volume; and
a tracking element adapted to provide an estimate of a position and/or orientation of the distal end of the catheter housing, said tracking element disposed within the catheter housing and immediately adjacent to the distal end.

8. The ultrasound catheter of claim 7, wherein the metal layer comprises copper, aluminum, gold, silver, or combinations thereof

9. The ultrasound catheter of claim 7 or claim 8, wherein the inner polymer tube and the outer polymer tube have sound velocities in the range 1.0 to 3.0 millimeters per microsecond, and acoustic impedances in the range of 1.0 to3.0 MegaRayls.

10. A method of manufacturing an ultrasound catheter housing comprising:
creating a polymer inner layer supported on an inner form or mandrel;
coating the polymer layer with metal;
adding a polymer outer layer over the metal layer; and
removing the inner form or mandrel.
